# EUROPEAN PATENT SPECIFICATION

(11) **EP 1 559 426 B1**
(45) Date of publication and mention of the grant of the patent: **09.02.2011**
(21) Application number: 02779994.9
(22) Date of filing: 05.11.2002
(51) Int. Cl.: A61K 31/4152, A61K 47/10, A61K 47/30, A61K 9/70, A61P 9/00

(54) **PERCUTANEOUS ABSORPTION PREPARATIONS CONTAINING 3-METHYL-1-PHENYL-2-PYRAZOLIN-5-ONE**
PERKUTANE ABSORPTIONSZUBEREITUNGEN MIT 3-METHYL-1-PHENYL-2-PYRAZOLIN-5-ON
PREPARATIONS D'ABSORPTION PERCUTANEE CONTENANT DU 3-METHYL-1-PHENYL-2-PYRAZOLIN-5-ONE

(43) Date of publication of application: 03.08.2005
(73) Proprietor: Lead Chemical Co. Ltd., Toyama-shi, Toyama-ken 930-0912 (JP)
(72) Inventor: MORI, Jun LEAD CHEMICAL CO., LTD., Toyama-shi, Toyama 930-0912 (JP); HORIUCHI, Tamaki LEAD CHEMICAL CO., LTD., Toyama-shi, Toyama 930-0912 (JP); YAMA, Seijiro LEAD CHEMICAL CO., LTD., Toyama-shi, Toyama 930-0912 (JP); WAKI, Hitomi LEAD CHEMICAL CO., LTD., Toyama-shi, Toyama 930-0912 (JP); SHIMADA, Shingo LEAD CHEMICAL CO., LTD., Toyama-shi, Toyama 930-0912 (JP); HASHITANI, Hitomi LEAD CHEMICAL CO., LTD., Toyama-shi, Toyama 930-0912 (JP)
(74) Representative: HOFFMANN EITLE
(86) International application number: PCT/JP2002/011518
(87) International publication number: WO 2004/041270

(56) References cited:
- EP-A- 0 739 626
- EP-A- 0 947 584
- EP-A- 0 974 350
- EP-A- 1 174 132
- WO-A1-02/00260
- JP-A- 3 127 727
- JP-A- 6 205 839
- JP-A- 10 265 373
- JP-A- 10 279 480
- JP-A- 62 063 512
- ZONDLO M M: "Final report on the safety assessment of phenyl methyl pyrazolone" 1992, JOURNAL OF THE AMERICAN COLLEGE OF TOXICOLOGY 1992 US, VOL. 11, NR. 4, PAGE(S) 475 - 488 , XP009111667 ISSN: 0730-0913 * the whole document *
- TANAKA MASAHIKO: "Pharmacological and clinical profile of the free radical scavenger edaravone as a neuroprotective agent" May 2002 (2002-05), FOLIA PHARMACOLOGICA JAPONICA, VOL. 119, NR. 5, PAGE(S) 301-308 , XP009111663 ISSN: 0015-5691 * abstract *

## Description

### FIELD OF THE INVENTION

The present invention relates to a 3-methyl-1-phenyl-2-pyrazolin-5-one percutaneous absorption preparation able to be used to protect brain functions in humans and improve and prevent cerebral dysfunction with respect to overall cerebral dysfunction including cerebral infarction and subarachnoid hemorrhage and the like, as well as to treat and prevent disorders such as arteriosclerosis, hepatic damage, renal damage, diabetes, and gastrointestinal mucous membrane damage.

### BACKGROUND OF THE INVENTION

3-methyl-1-phenyl-2-pyrazolin-5-one is a brain protecting drug which has a free radical eliminating function and is used with an injectable solution (intravenous drip infusion; intravenous injection by drip) as an improving drug for neurological syndromes, impairment of daily living activities, and functional impairment in humans. Recently, many people are suffering from cerebral dysfunction brought on by aging, variation in diet, an increase in stress in daily life, and the like, and as a result, quick and precise countermeasures for cerebral dysfunction is now an important issue in medicine.

Free radicals such as hydroxy radicals (OH) which are produced in excess in the body during ischemia due to cerebral infarction or the like and after restoration of blood flow thereafter result in a chain reaction of oxygenation damage in cell membranes in humans, thereby further worsening cerebral ischemia damage. In this case, if an injectable solution containing 3-methyl-1-phenyl-2-pyrazolin-5-one (proprietary name: RADICUT inj. 30mg; injectable solution including 30mg of active ingredient per dose) is used, the injectable solution demonstrates excellent treatment effects against cerebral ischemia damage by eliminating the hydroxy radicals in the bodies of humans.

The injectable solution, however, causes a patient pain due to the fact that an injection needle punctures the body (vein) of the patient during intravenous drip infusion of the injectable solution. Further, the intravenous drip infusion is normally performed on a patient who is lying down on a bed, which means the patient is restricted to the bed for a certain period of time (i.e., while the intravenous drip infusion is being performed). In addition, with the exception of some injectable solutions such as insulin and interferon, injections are not able to be administered by the patient him/herself. An intravenous drip infusion of the injectable solution containing 3-methyl-1-phenyl-2-pyrazolin-5-one is also obviously not able to be administered by the patient him/herself, which means that (administration of) the intravenous drip infusion must be done by a doctor, a female nurse, or a male nurse. As a result, the patient is compelled to be admitted into a hospital or go to a hospital for the intravenous drip infusion. Even if the patient is admitted into a hospital or goes to a hospital for the intravenous drip infusion, the patient feels pain during the intravenous drip infusion, and a healthcare practitioner such as a doctor, female nurse, or male nurse must take the time to perform (administer) the intravenous drip infusion. As a result, if the intravenous drip infusion is administered twice daily according to the dosage regimen, for example, each time it causes the patient pain and takes the time of a healthcare practitioner.

Also, side-effects such as impaired liver function are being reported following intravenous drip infusion of the injectable solution. A temporary increase in the concentration of medication in the blood from the intravenous drip infusion can easily be assumed to be one cause. Given these circumstances, development of a preparation containing 3-methyl-1-phenyl-2-pyrazolin-5-one which is easy to administer, which has lasting effects over an extended period of time, and which has few side-effects is desirable.

When 3-methyl-1-phenyl-2-pyrazolin-5-one is used as an agent to normalize brain function, methods of administration as an injectable solution, an oral agent, or a suppository (administered rectally) are disclosed in Japanese Patent Application Laid-Open No. 61-263917, for example, but only an injectable solution is currently used in clinical practice. One reason for this is because many disorders that the agent to normalize brain function is aimed to treat occur following cerebral infarction, so many patients to whom the agent to normalize brain function is administered are incapacitated or unconscious which makes it difficult to administer the agent orally. In addition, many of the patients to whom the agent to normalize brain function is administered are elderly patients who fundamentally have a difficult time taking medication orally.

Furthermore, 3-methyl-1-phenyl-2-pyrazolin-5-one is quickly metabolized in the liver by glucuronate conjugation or sulfate conjugation so the effectiveness from the first passage effect in the liver is extremely low when administered orally. Also, 3-methyl-1-phenyl-2-pyrazolin-5-one is disclosed as a lipid peroxide production inhibiting agent (see Japanese Patent Application Laid-Open No. 62-108814, for example), an antiulcer agent (see Japanese Patent Application Laid-Open No. 3-215425, for example), an anti-hyperglycemic agent (see Japanese Patent Application Laid-Open No. 3-215426, for example), an agent for ocular disease (see Japanese Patent Application Laid-Open No. 7-25765, for example), an agent for treating/preventing acute hepatic failure (see Japanese Patent Application Laid-Open No. 9-52831, for example), and the like. All of these agents, however, are administered orally, intravenously, or rectally and thus have the drawbacks described above in clinical practice.
The safety assessment of phenyl methyl pyrazolin (PMP) is described in the Journal of the American College of Toxicology, vol. 11, no. 4, 1992. External preparations containing a variety of active ingredients other than PMP are reported in EP 0 974 350 A1, EP 0 947 584 A1, EP 0 739 626 A2, EP 1 174 132 A1, JP 10265373 A, JP 62063512 A, JP 6205839 and JP 03127727. The medical use of PMP is described in XP 009111663, JP 10279480 and WO 02/00260 A1.

### DISCLOSURE OF THE INVENTION

In view of the current situation, the inventors have achieved an invention of a percutaneous absorption preparation containing 3-methyl-1-phenyl-2-pyrazolin-5-one which has a medicinal effect equal to, or greater than, the medicinal effect of 3-methyl-1-phenyl-2-pyrazolin-5-one when used as an injectable solution, and which solves the problems of the related art by employing a form of the percutaneous absorption preparation (also including a form of percutaneous absorption adhesive preparation) as a form of preparation instead of an injectable solution, as a result of intensive study of other methods of administration, aside from the foregoing method of administration, for 3-methyl-1-phenyl-2-pyrazolin-5-one.

The present invention relates to a percutaneous absorption preparation containing 3-methyl-1-phenyl-2-pyrazolin-5-one, characterised in that it contains, as an active ingredient, 0.1 to 30 percent by mass of 3-methyl-1-phenyl-2-pyrazolin-5-one represented by the following formula: 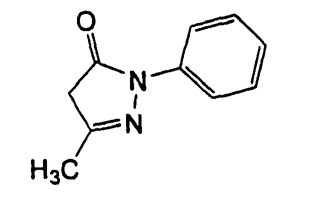 or a medically acceptable salt thereof in an aqueous base, wherein the aqueous base contains, based on a total amount of the aqueous base, 1 to 20 percent by mass of a water-soluble polymer, 0.01 to 20 percent by mass of a cross-linking agent, 10 to 80 percent by mass of polyhydric alcohol, and 1 to 80 percent by mass of water.

The present invention also relates to a percutaneous absorption adhesive preparation containing 3-methyl-1-phenyl-2-pyrazolin-5-one, characterized in that a support medium, a base layer formed of an aqueous base containing, as an active ingredient, 0.1 to 30 percent by mass of 3-methyl-1-phenyl-2-pyrazolin-5-one represented by the following formula: 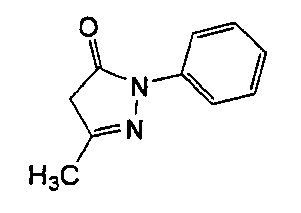 or a medically acceptable salt thereof, and a liner are sequentially laminated and formed, wherein the aqueous base contains, based on a total amount of the aqueous base, 1 to 20 percent by mass of a water-soluble polymer, 0.01 to 20 percent by mass of a cross-linking agent, 10 to 80 percent by mass of polyhydric alcohol, and 1 to 80 percent by mass of water.

The present invention furthermore relates to a percutaneous absorption preparation containing 3-methyl-1-phenyl-2-pyrazolin-5-one, characterised in that it contains, as an active ingredient, 0.1 to 30 percent by mass of 3-methyl-1-phenyl-2-pyrazolin-5-one represented by the following formula: 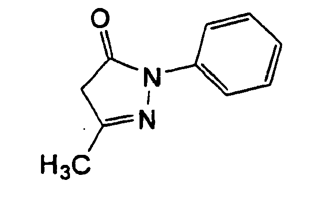 or a medically acceptable salt thereof in a rubber base, wherein the rubber base contains, based on the total amount of the rubber base, 10 to 50 percent by mass of a rubber polymer, 10 to 50 percent of a plasticizer, and 5 to 50 percent by mass of a tackifier.

Furthermore, the present invention is directed to a percutaneous absorption adhesive preparation containing 3-methyl-1-phenyl-2-pyrazolin-5-onecharacterized in that a support medium, a base layer formed of a rubber base containing, as an active ingredient, 0.1 to 30 percent by mass of 3-methyl-1-phenyl-2-pyrazolin-5-one represented by the following formula: 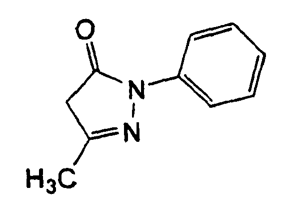 or a medically acceptable salt thereof, and a liner are sequentially laminated and formed, wherein the rubber base contains, based on the total amount of the rubber base, 10 to 50 percent by mass of a rubber polymer, 10 to 50 percent of a plasticizer, and 5 to 50 percent by mass of a tackifier.

These preparations have the following advantages.
a) If this preparation is used, it is easy to administer, as well as stop the administration of, 3-methyl-1-phenyl-2-pyrawlin-5-one (the agent). More specifically, for example, when there are side effects from the agent, administration of the agent can be discontinued simply by wiping off the preparation (when it is in the form of an ointment, for example), or removing the preparation (when it is in the form of an adhesive preparation, for example).
b) Using a specific amount of the preparation (applying it to the skin, for example) (when it is in the form of an ointment, for example) or using the adhesive preparation (by adhering it to the skin, for example) makes it is possible to maintain an effective concentration of the medication in the blood over an extended period of time.
c) Because an effective concentration of the agent is able to be maintained in the blood over an extended period of time when the preparation is used once, the number of times the medication is administered can be reduced compared with the conventional intravenous drip infusion. As a result, it is possible to promote patient compliance and reduce the load on the caretaker during treatment.
d) While the preparation is being used, the concentration of the medication in the blood is maintained within a predetermined range so the concentration of the medication in the blood will not temporarily increase to an undesirable value as is the case with the intravenous drip infusion. Accordingly, it is possible to avoid the side-effects from the medication which accompany a temporary increase in the concentration of the medication in the blood.

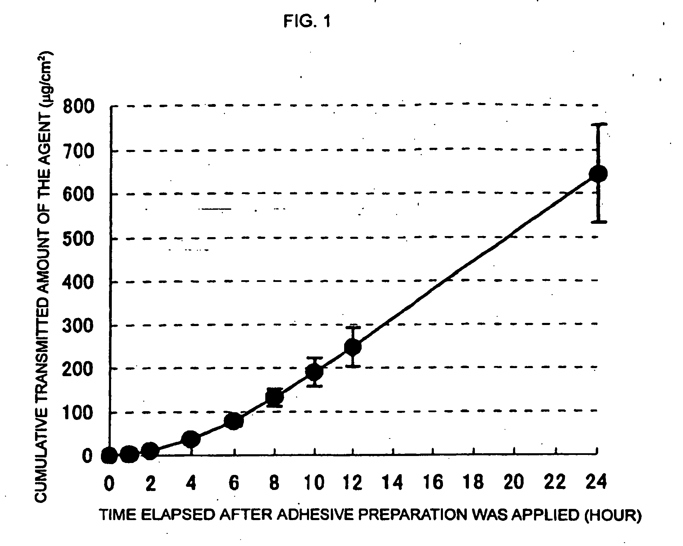

FIG. 1 is a graph showing the relationship between time elapsed after the adhesive preparation was applied and the cumulative transmitted amount of the agent (3-methyl-1-phenyl-2-pyrazolin-5-one) in a case where the adhesive preparation according to a first embodiment has been applied to the skin of a rat.

### DETAILED DESCRIPTION OF THE EMBODIMENT

Hereinafter, the preparation and adhesive preparation will be described.

In the preparation and the adhesive preparation, 3-methyl-1-phenyl-2-pyrazolin-5-one, which is the active ingredient, differs according to the formula, but it is desirable that 0.1 to 30 percent by mass, and more preferably 0.5 to 20 percent by mass, in particular 0.5 to 10 percent by mass, based on the total amount of base, be blended in a suitable base.

The preparation may be in various suitable forms, for example, a solution, a slurry, an ointment, a paste, rubber, or the like, and can be manufactured as it is or in a more suitable form.

If the preparation is used in the form of an adhesive preparation, it can be applied to the skin in that form which makes it convenient and easy to use. The adhesive preparation may also be in various adhesive preparation forms such as, for example, an adhesive skin patch, a plaster agent, a tape agent, or the like, depending on the use.

The adhesive preparation can be manufactured by, for example, adding a predetermined amount of 3-methyl-1-phenyl-2-pyrazolin-5-one in a form suitable for coating (such as ointment form) to a suitable base (such as an aqueous base or a rubber base), applying this at a predetermined thickness to a suitable support medium, covering it from above with a predetermined liner, and cutting it to the desired size. Depending on the manufacturing method, the adhesive preparation may also be formed by, for example, first coating a liner with a base containing 3-methyl-1-phenyl-2-pyrazolin-5-one to form a base layer, covering this from above with a support medium, and then transferring the base layer onto the support medium.

As the aqueous base or the rubber base, an aqueous base made of a mixture of the following components, for example, can be used.
I. Aqueous base
   Component 1) : water-soluble polymer
   Component 2) : cross-linking agent
   Component 3) : polyhydric alcohol
II. Rubber base
   Component 4) : rubber polymer
   Component 5) : plasticizer
   Component 6) : tackifier

The components 1) to 6) will be hereinafter be described.

Examples of the water-soluble polymer of component 1) include polyacrylic acid, polyacrylate, polyacrylic acid moiety neutralizer, polyacrylamide, polyethylene imine, polyvinyl alcohol, polyvinylpyrrolidone, carboxy vinyl polymer, methylcellulose, carboxymethylcellulose, carboxymethylcellulose sodium, hydroxyethylcellulose, starch acrylate, ethyl vinyl acetate, gelatin, starch, Eudragid, alginic acid, sodium alginate, tragacanth, and the like. Only one type of water-soluble polymer may be used or two or more types of water-soluble polymers may be suitably mixed together at a predetermined ratio and used. The compound content of the water-soluble polymer is 1 to 20 percent by mass, and preferably 3 to 6 percent by mass, based on the total amount of the water-soluble base.

As the cross-linking agent of component 2), for example, salts can be used which produce bivalent or trivalent metal ions when dissolved in water or the like. Examples of the cross-linking agent include hydroxides such as aluminum hydroxide and magnesium aluminum hydroxide, or inorganic acid or organic acid salts such as aluminum chloride, aluminum sulfate, dihydroxyaluminum aminoacetate, kaolin, aluminum stearate, magnesium hydroxide, magnesium chloride, and magnesium sulfate, or an aluminate such as sodium aluminate, inorganic aluminum complex salt and organic aluminum chelate compound, synthetic hydrotalcite, magnesium aluminometasilicate, magnesium aluminosilicate, aluminum nitrate, aluminum sulfate, EDTA-aluminum, aluminum allantoinate, aluminum acetate, aluminum glycinal and the like. Only one type of cross-linking agent may be used or two or more types of cross-linking agents may be suitably mixed together at a predetermined ratio and used. The compound content of the cross-linking agent is 0.01 to 20 percent by mass, and more preferably 0.1 to 10 percent by mass, based on the total amount of the water-soluble base.

The salts that produce bivalent or trivalent metal ions and which serve as the cross-linking agent may be readily soluble in water or may be very insoluble in water. When an aluminum compound that is very insoluble in water is used as the cross-linking agent, a reaction speed adjuster can be added to the reaction system in order to facilitate gelatinization. In particular, the addition of acid makes it possible to increase the reaction speed of the gelatinization. The gelatinization reaction speeds up remarkably by adding an organic acid that includes a hydroxyl group or a salt thereof, in particular, as the acid. Examples of the reaction speed adjuster include organic bases, organic acid salts, and organic acids having a chelate forming ability or coordinating property with respect to metal ions, such as citric acid, lactic acid, tartaric acid, gluconic acid, glycolic acid, malic acid, fumaric acid, meta sulfonic acid, maleic acid, acetic acid, EDTA di sodium, urea, triethylamine, ammonia, and inorganic acids such as hydrochloric acid, phosphoric acid, sulfuric acid, nitric acid and hydrobromic acid.

Examples of the polyhydric alcohol of component 3) include ethylene glycol, propylene glycol, trimethylene glycol, 1, 3-butanediol, ethylene glycol monobutyl ether, triethyleneglycol, 1, 4-butanediol, glycerin, trioxyisobutane, erythritol, pentaerythritol, xylitol, adonite, arodulcite, sorbitol, sorbit solution, mannitol, and polyethyleneglycol. Only one type of polyhydric alcohol may be used or two or more types of polyhydric alcohols may be suitably mixed together at a predetermined ratio and used. The compound content of the polyhydric alcohol is 10 to 80 percent by mass, and more preferably 10 to 60 percent by mass, based on the total amount of the base.

Examples of the rubber polymer of component 4) include a styreneisoprene-styrene block copolymer, a styrene butadiene block copolymer, polyisobutylene, crude caoutchouc, polyisoprene, polybutene and the like. Only one type of rubber polymer may be used or two or more types of rubber polymers may be suitably mixed together at a predetermined ratio and used. The compound content of the rubber polymer is 10 to 70 percent by mass, and more preferably 20 to 50 percent by mass, based on the total amount of the base.

Examples of the plasticizer of component 5) include liquid paraffin, vegetable oil, animal oil, polybutene, low-molecular weight polyisobutylene, petrolatum, lanoline, premium aliphatic ester, and the like. Only one type of plasticizer may be used or two or more types of plasticizers may be suitably mixed together at a predetermined ratio and used. The compound content of the plasticizer is 10 to 70 percent by mass, and more preferably 20 to 50 percent by mass, based on the total amount of the base.

Examples of the tackifier of component 6) include petroleum resin, a rosin resin, hydrogenated rosin, ester gum, terpene resin, modified terpene resin, aromatic hydrocarbon resin, aliphatic hydrocarbon resin, and the like. Only one type of tackifier may be used or two or more types of tackifiers may be suitably mixed together at a predetermined ratio and used. The compound content of the tackifier is 5 to 50 percent by mass, and more preferably 10 to 30 percent by mass, based on the total amount of the base.

The support member used in the percutaneous absorption preparation of the present invention is not particularly limited, and a common material can be used for the support member of the percutaneous absorption preparation. For example, the support member may be a natural or synthetic polymer woven fabric, non-woven fabric, sheet, film, or a laminated body thereof. Preferable examples of the synthetic polymer include polyvinylchloride resin, a polyethylene resin (such as polyethylene resin or a blend of polyethylene resin and another resin), an ethylene copolymer (such as a copolymer of ethylene and another monomer), a polypropylene resin (such as polypropylene resin or a blend of polypropylene resin and another resin), polyurethane resin, and the like. The size, shape, thickness and the like of the support member is suitably selected.

The liner used in the percutaneous absorption adhesive preparation of the present invention is not particularly limited, and a common material can be used for the liner of the percutaneous absorption preparation. For example, the liner may be a natural or synthetic polymer sheet, film, or a laminated body thereof. Preferable examples of the liner include release paper that has been treated (synthetic polymer coated, for example) to facilitate release, and sheets, films, or a laminated body thereof, of cellophane, polyethylene, polyethylene terephthalate, polypropylene, polyester, polyvinylidene chloride.

As a base layer in the percutaneous absorption adhesive preparation of the present invention, a base layer can be used in which a base containing, if necessary, a predetermined content of the components 1) to 6), for example, in addition to containing 0.1 to 30 percent by mass of 3-methyl-1-phenyl-2-pyrazolin-5-one or a medically acceptable salt thereof, just like the preparation, is formed in a layer of a predetermined thickness.

In this preparation or this adhesive preparation, in addition to the necessary component (the effective ingredient being 0.1 to 30 percent by mass of 3-methyl-1-phenyl-2-pyrazolin-5-one or a medically acceptable salt thereof) and the component 1) to component 6) or the like, various agents commonly used in conventional percutaneous absorption preparations or percutaneous absorption adhesive preparations can be blended together at predetermined ratios as necessary. Some of these various agents include a percutaneous absorption accelerator, a tackifier, a softener, an antioxidant, an age resistor, a preservative, an aromatizing agent, a pH adjuster, an emulsifying agent, a dispersing agent, a stabilizing agent, an antiseptic agent, a diluting agent, and a dissolving agent.

The effective ingredient 3-methyl-1-phenyl-2-pyrazolin-5-one or a medically acceptable salt thereof is highly reactive because it has a free radical eliminating function. As a result, its stability is poor. In an injectable solution, the number of base components is low so the stability of the effective ingredient when used in an injectable solution is good. In a percutaneous absorption preparation or a percutaneous absorption adhesive preparation, however, other additives are often added in addition to the necessary component during manufacturing. Therefore, when the effective ingredient is used in a percutaneous absorption preparation or a percutaneous absorption adhesive preparation, the stability of the effective ingredient decreases depending on the formula. In this case, adding an antioxidant as a stabilizing agent is effective to stabilize the product.

Examples of the antioxidant include ascorbic acid, palmitic acid, acid sodium sulfite, edetate sodium, edetate tetrasodium, dried sodium sulfite, citric acid, sodium acid citrate, tocopheryl acetate, dl-α-tocopherol, potassium dichloro isocyanuric acid, dibutyl hydroxytoluene, butylhydroxyanisol, soybean lecithin, sodium pyrosulfite, 1, 3-butylene glycol, benzotriazole, penta-erythrol-tetrakis [3-(3, 5-di-tertiery butyl-4-hydroxyphenyl) propionate], propyl gallate, 2-mercaptobenzimidazole, and the like. Only one type of the antioxidant may be used or two or more types of antioxidants may be suitably mixed together at a predetermined ratio and used. The compound content of the antioxidant is 0.005 to 20 percent by mass, and preferably 0.1 to 5 percent by mass, based on the total amount of the base.

The percutaneous absorption accelerator is not particularly limited as long as it is one which is normally used in percutaneous absorption preparations. Examples of the percutaneous absorption accelerator include alcohol, fatty acid, fatty ester, fatty ether, lactic acid ester, acetic ester, terpene compound, pyrrolidone derivative, organic acid, organic acid ester, essential oil, hydrocarbon, azone or a derivative thereof, and the like. More specifically, the percutaneous absorption accelerator is ethanol, oleyl alcohol, lauryl alcohol, myristyl alcohol, cetyl alcohol, crotamiton, cyclodextrin, calcium thioglycollate, N-methyl-2-pyrrolidone, ethyl lactate, cetyl lactate, lactic acid, urea, 1-menthol, mentha oil, d-limonene, d1-camphor, or the like. Only one type of percutaneous absorption accelerator may be used or two or more types of percutaneous absorption accelerators may be suitably mixed together at a predetermined ratio and used. The compound content of the percutaneous absorption accelerator is 0.1 to 20 percent by mass, and preferably 0.1 to 5 percent by mass, based on the total amount of the base.

Examples of the dissolving agent include n-methyl-2-pyrrolidone, crotamiton, macrogol, isopropanol, mentha oil, propylene glycol, butylene glycol, oleyl alcohol, isopropyl myristate, and the like. n-methyl-2-pyrrolidone and crotamiton are particularly effective as dissolving agents due to the high solubility of 3-methyl-1-phenyl-2-pyrazolin-5-one.

### (Examples)

Examples of the present invention will hereinafter be described.

### Example 1

Liquid A was adjusted by mixing 5 parts sodium polyacrylate, 6 parts starch acrylate, 12 parts talc, and 29.1 parts concentrated glycerin. Liquid B was adjusted by dissolving 2.3 parts tartaric acid in 30 parts water. Liquid C was adjusted by dissolving 3 parts 3-methyl-1-phenyl-2-pyrazolin-5-one in 8 parts n-methyl-2-pyrrolidone and 2 parts crotamiton. Liquid B and liquid C were added to liquid A. Also, 2.5 parts methyl acrylate/acrylic acid 2-ethylhexyl copolymer resin emulsion and 0.1 parts aluminum hydroxide gel were added and mixed evenly. A base layer was then formed by spreading this mixture (this preparation) at a predetermined thickness on a polyester non-woven fabric (support medium) of predetermined dimensions (length dimensions x width dimensions x thickness; the same applies hereafter). This base layer was then covered with a polyethylene film (liner) of predetermined dimensions. This was then cut into predetermined dimensions to obtain a percutaneous absorption adhesive preparation containing 3-methyl-1-phenyl-2-pyrazolin-5-one of Example 1.

### Example 2

20 parts polybutene, 10 parts polyisobutylene, 25 parts styrene-isobutylene-styrene block copolymer, 0.5 parts di-butylhydroxytoluene, 14.5 parts liquid paraffin, 10 parts water absorbing-polymer [starch acrylate 1000 (proprietary name: Sunwet IM 1000)], 17 parts adhesive (proprietary name: Alcon P-100), and 3 parts 3-methyl-1-phenyl-2-pyrazolin-5-one were dissolved in 60 parts isohexane. Then, a base layer was formed by spreading this solution (the preparation) at a predetermined thickness on a polyvinyl chloride sheet (support member) of predetermined dimensions. This was then dried and the solvent removed, after which the base layer was covered with a polyester film (liner). This was then cut into predetermined dimensions to obtain a percutaneous absorption adhesive preparation containing 3-methyl-1-phenyl-2-pyrazolin-5-one of Example 2.

### Example 3

Liquid A was adjusted by mixing, at 140 degrees Celsius, 20 parts polybutene, 10 parts polyisobutylene, 19.5 parts liquid paraffin, 25 parts styrene-isobutylene-styrene block copolymer, 0.5 parts di-butylhydroxytoluene, and 17 parts adhesive (proprietary name: Alcon P-100). Liquid B was adjusted by evenly mixing 3 parts 3-methyl-1-phenyl-2-pyrazolin-5-one into 5 parts propylene glycol. After heating liquid A from room temperature to 120 degrees Celsius, liquid B was then added to, and mixed with, liquid A. A base layer was then formed by spreading this mixture (this preparation) at a predetermined thickness on a polyester non-woven fabric of predetermined dimensions. This base layer was then covered with a polyethylene film (liner) of predetermined dimensions. After being cooled to room temperature, this was then cut into predetermined dimensions to obtain a percutaneous absorption adhesive preparation containing 3-methyl-1-phenyl-2-pyrazolin-5-one of Example 3.

### Test Example 1: in vitro skin transmission test

### 1) Test method

The following test was conducted using three samples (number of samples: 3). Under pentobarbital anesthesia, the hair on the abdomens of male Wistar rats (7 weeks old) was shaved off, skin was excised, and fat on the dermal side of the skin was carefully removed. The skin was attached, with the dermal side down, to a vertical diffusion cell through which water at 37 degrees Celsius had been circulated in advance. A 1 cm in diameter cutout of the adhesive preparation of Example 1 was applied to the center portion thereof and then pinch-fixed using a dome-shaped cell. Next, a receiver liquid that had been held in a constant temperature bath of 37 degrees Celsius was applied to the dermal side, an L-shaped tube was fixed with fixing Parafilm to a sampling port, and then a fixed amount of receiver solution was added. The receiver solution was agitated with a magnetic stirrer, after which a fixed amount of the receiver solution was regularly collected and the same amount of receiver liquid was replenished. The quantity of the agent (3-methyl-1-phenyl-2-pyrazolin-5-one) in the collected sample was made a determined quantity by high-performance liquid chromatography (HPLC) and the amount of 3-methyl-1-phenyl-2-pyrazolin-5-one transmitted through the skin was then calculated.

### 2) Result

The relationship between the time elapsed after application of the adhesive preparation of Example 1 and the cumulative transmitted amount of the agent (3-methyl-1-phenyl-2-pyrazolin-5-one) is illustrated in Table 1 and FIG. 1.

**Table 1: Relationship between time elapsed after application of adhesive preparation and cumulative transmitted amount of the agent**

| Time Elapsed (hour) | Cumulative Transmitted Amount | Standard Deviation |
|---|---|---|
| | (µg/cm²) | |
| 0 | 0 | 0 |
| 1 | 3.41 | 0.23 |
| 2 | 10.52 | 1.04 |
| 4 | 35.79 | 7.22 |
| 6 | 78.15 | 11.30 |
| 8 | 131.03 | 20.75 |
| 10 | 190.34 | 33.25 |
| 12 | 247.59 | 44.28 |
| 24 | 643.53 | 111.28 |

As is evident from Table 1 and FIG. 1, the adhesive preparation of Example 1 shows good skin transmission. The amount of the agent (3-methyl-1-phenyl-2-pyrazolin-5-one) transmitted through the skin (the cumulative transmitted amount of the agent) increases over time (it is almost directly proportionate to time after a predetermined period of time has passed) for up to 24 hours.

### Test Example 2: Rabbit primary skin irritation test

### 1) Test method

A primary skin irritation test was conducted using a male JW/CSK rabbit (12 weeks), the hair of which was shaved off of a back portion the day before the test. Evaluation was done using the primary irritation index according to the Draize method. A section of healthy skin and a section of damaged skin that had been scratched with a sterilized injection needle were established and the test was conducted regarding the irritation of the skin of those sections. The application area of the adhesive preparation was set at a size of 5 cm². One adhesive preparation each of Examples 1, 2, and 3 was applied to the section of healthy skin and the section of damaged skin. Twenty-four hours after being applied, the adhesive preparations of Examples 1, 2, and 3 were removed from the section of healthy skin and the section of damaged skin. One hour, 24 hours, and 48 hours after the adhesive preparations were removed, erythema, edema, and scab-formation in the sections was determined according to the criterion of the Draize method. From the results, a skin irritation index (PII) was calculated and evaluated following the evaluation classifications.

Also, as a control, evaluations were also conducted by a test method similar to the method described above for an adhesive preparation (Control 1) which was adjusted by coating 0.25g of an ointment containing 5 percent by mass of sodium lauryl sulfate and 95 percent by mass of white petrolatum onto a non-woven fabric, and an adhesive preparation (Control 2) which was adjusted by using a preparation of the composition of Example 1 excluding the agent (3-methyl-1-phenyl-2-pyrazolin-5-one).

### 2) Result

The results are shown in Table 2 below.

**Table 2: Rabbit primary skin irradiation test**

| Adhesive Preparation | Skin Irritation Index (P.I.I.) | Safety Classification |
|---|---|---|
| Example 1 | 0.06 | Weak Irritant |
| Example 2 | 0.10 | Weak Irritant |
| Example 3 | 0.10 | Weak Irritant |
| Control 1 | 6.80 | Strong Irritant |
| Control 2 | 0.06 | Weak Irritant |

The adhesive preparation of Examples 1, 2, and 3 is in the range of a weak irritant according to the criterion of the Draize method, just like the adhesive preparation of comparison 2 which does not contain the agent (3-methyl-1-phenyl-2-pyrazolin-5-one). Therefore, it is evident that this adhesive preparation is a percutaneous absorption preparation which causes extremely little skin irritation.

### INDUSTRIAL APPLICABILITY

The percutaneous absorption preparation according to the present invention causes little skin irritation, and, by applying it to the skin of an individual in the form of a percutaneous absorption adhesive preparation, for example, the agent (3-methyl-1-phenyl-2-pyrazolin-5-one), which is an effective ingredient against cerebral dysfunction, can be maintained at an effective concentration over an extended period of time, and can be easily administered by an individual.

The method of use (starting and stopping administration of the agent) of the preparation or the adhesive preparation is easy, and during use, the agent is gradually absorbed through the skin. As a result, the agent is able to be effective over a long period of time without causing a temporary increase in the concentration of the agent in the blood.

Further, the preparation or adhesive preparation does not cause the patient pain or restrict the patient for a certain period of time during use as does the conventional injectable solution (intravenous drip infusion).

Accordingly, the preparation or adhesive preparation is useful for protecting brain functions in humans and improving and preventing cerebral dysfunction with respect to overall cerebral dysfunction including cerebral infarction and subarachnoid hemorrhage and the like, as well as treating and preventing disorders such as arteriosclerosis, hepatic damage, renal damage, diabetes, and gastrointestinal mucous membrane damage.

## Claims

1. A percutaneous absorption preparation containing 3-methyl-1-phenyl-2-pyrazolin-5-one, **characterised in that** it contains, as an active ingredient, 0.1 to 30 percent by mass of 3-methyl-1-phenyl-2-pyrazolin-5-one represented by the following formula: 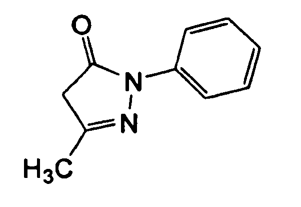 or a medically acceptable salt thereof in an aqueous base, wherein the aqueous base contains, based on a total amount of the aqueous base, 1 to 20 percent by mass of a water-soluble polymer, 0.01 to 20 percent by mass of a cross-linking agent, 10 to 80 percent by mass of polyhydric alcohol, and 1 to 80 percent by mass of water.

2. A percutaneous absorption adhesive preparation containing 3-methyl-1-phenyl-2-pyrazolin-5-one, **characterized in that** a support medium, a base layer formed of an aqueous base containing, as an active ingredient, 0.1 to 30 percent by mass of 3-methyl-1-phenyl-2-pyrazolin-5-one represented by the following formula: 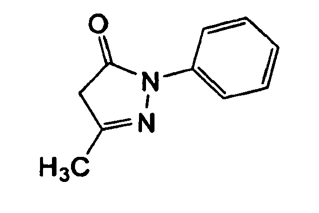 or a medically acceptable salt thereof, and a liner are sequentially laminated and formed, wherein the aqueous base contains, based on a total amount of the aqueous base, 1 to 20 percent by mass of a water-soluble polymer, 0.01 to 20 percent by mass of a cross-linking agent, 10 to 80 percent by mass of polyhydric alcohol, and 1 to 80 percent by mass of water.

3. A percutaneous absorption preparation containing 3-methyl-1-phenyl-2-pyrazolin-5-one, **characterised in that** it contains, as an active ingredient, 0.1 to 30 percent by mass of 3-methyl-1-phenyl-2-pyrazolin-5-one represented by the following formula: 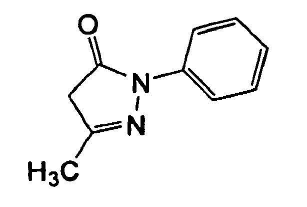 or a medically acceptable salt thereof in a rubber base, wherein the rubber base contains, based on the total amount of the rubber base, 10 to 50 percent by mass of a rubber polymer, 10 to 50 percent of a plasticizer, and 5 to 50 percent by mass of a tackifier.

4. A percutaneous absorption adhesive preparation containing 3-methyl-1-phenyl-2-pyrazolin-5-one, **characterized in that** a support medium, a base layer formed of a rubber base containing, as an active ingredient, 0.1 to 30 percent by mass of 3-methyl-1-phenyl-2-pyrazolin-5-one represented by the following formula: 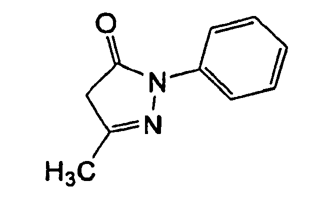 or a medically acceptable salt thereof, and a liner are sequentially laminated and formed, wherein the rubber base contains, based on the total amount of the rubber base, 10 to 50 percent by mass of a rubber polymer, 10 to 50 percent of a plasticizer, and 5 to 50 percent by mass of a tackifier.

5. The percutaneous absorption preparation according to claim 1 or 2, **characterized in that** the cross-linking agent is aluminium hydroxide.

6. The percutaneous preparation according to any of claims 1 to 4, **characterized in that** the preparation further contains N-methyl-2-pyrrolidone as a dissolving agent.

7. The percutaneous absorption preparation according to any of claims 1 to 4, **characterized in that** the preparation further contains crotamiton as a percutaneous absorption accelerator.

8. The preparation according to any of claims 1 to 7 for use in the protection of brain functions.

9. The preparation according to any one of claims 1 to 7 for use in the improvement and prevention of cerebral dysfunction, including cerebral infarction and subarachnoid hemorrhage.

10. The preparation according to any one of claims 1 to 7 for use in the improvement and prevention of arteriosclerosis, hepatic damage, renal damage, diabetes and gastrointestinal mucous membrane damage.

## Patentansprüche

1. Perkutane Resorptionszubereitung, enthaltend 3-Methyl-1-phenyl-2-pyrazolin-5-on, **dadurch gekennzeichnet, dass** sie als aktiven Bestandteil 0,1 bis 30 Masse-% 3-Methyl-1-phenyl-2-pyrazolin-5-on der folgenden Formel: 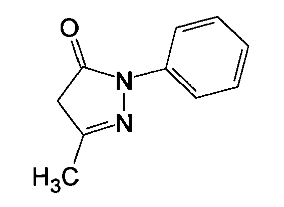 oder ein medizinisch annehmbares Salz davon in einer wässrigen Basis enthält, wobei die wässrige Basis, bezogen auf die Gesamtmenge der wässrigen Basis, 1 bis 20 Masse-% eines wasserlöslichen Polymers, 0,01 bis 20 Masse-% eines Vernetzungsmittels, 10 bis 80 Masse-% eines mehrwertigen Alkohols und 1 bis 80 Masse-% Wasser enthält.

2. Perkutane, anhaftende Resorptionszubereitung, enthaltend 3-Methyl-1-phenyl-2-pyrazolin-5-on, **dadurch gekennzeichnet, dass** ein Trägermedium, eine Basisschicht, die aus einer wässrigen Basis, enthaltend als aktiven Bestandteil 0,1 bis 30 Masse-% 3-Methyl-1-phenyl-2-pyrazolin-5-on der folgenden Formel: 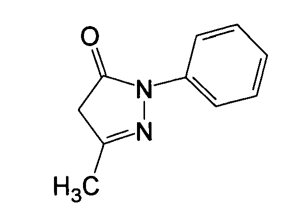 gebildet ist, oder ein medizinisch annehmbares Salz davon und eine Decklage aufeinanderfolgend laminiert und geformt werden, wobei die wässrige Basis, bezogen auf die Gesamtmenge der wässrigen Basis, 1 bis 20 Masse-% eines wasserlöslichen Polymers, 0,01 bis 20 Masse-% eines Vernetzungsmittels, 10 bis 80 Masse-% eines mehrwertigen Alkohols und 1 bis 80 Masse-% Wasser enthält.

3. Perkutane Resorptionszubereitung, enthaltend 3-Methyl-1-phenyl-2-pyrazolin-5-on, **dadurch gekennzeichnet, dass** sie als aktiven Bestandteil 0,1 bis 30 Masse-% 3-Methyl-1-phenyl-2-pyrazolin-5-on der folgenden Formel: 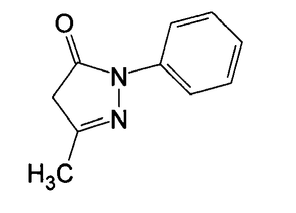 oder ein medizinisch annehmbares Salz davon in einer Kautschukbasis enthält, wobei die Kautschukbasis, bezogen auf die Gesamtmenge der Kautschukbasis, 10 bis 50 Masse-% eines Kautschukpolymers, 10 bis 50 Masse-% eines Weichmachers und 5 bis 50 Masse-% eines klebrigmachenden Mittels enthält.

4. Perkutane, anhaftende Resorptionszubereitung, enthaltend 3-Methyl-1-phenyl-2-pyrazolin-5-on, **dadurch gekennzeichnet, dass** ein Trägermedium, eine Basisschicht, die aus einer Kautschukbasis, enthaltend als aktiven Bestandteil 0,1 bis 30 Masse-% 3-Methyl-1-phenyl-2-pyrazolin-5-on der folgenden Formel: 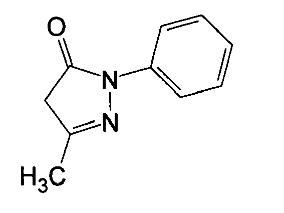 gebildet ist, oder ein medizinisch annehmbares Salz davon und eine Decklage aufeinanderfolgend laminiert und geformt werden, wobei die Kautschukbasis, bezogen auf die Gesamtmenge der Kautschukbasis, 10 bis 50 Masse-% eines Kautschukpolymers, 10 bis 50 Masse-% eines Weichmachers und 5 bis 50 Masse-% eines klebrigmachenden Mittels enthält.

5. Perkutane Resorptionszubereitung gemäss Anspruch 1 oder 2, **dadurch gekennzeichnet, dass** das Vernetzungsmittel Aluminiumhydroxid ist.

6. Perkutane Zubereitung gemäss irgendeinem der Ansprüche 1 bis 4, **dadurch gekennzeichnet, dass** die Zubereitung ferner N-Methyl-2-pyrrolidon als Lösungsmittel enthält.

7. Perkutane Resorptionszubereitung gemäss irgendeinem der Ansprüche 1 bis 4, **dadurch gekennzeichnet, dass** die Zubereitung ferner Crotamiton als perkutanen Resorptionsbeschleuniger enthält.

8. Zubereitung gemäss irgendeinem der Ansprüche 1 bis 7 zur Verwendung beim Schutz der Gehirnfunktionen.

9. Zubereitung gemäss irgendeinem der Ansprüche 1 bis 7 zur Verwendung bei der Verbesserung und Prävention von Hirnfunktionsstörungen, einschliesslich Hirninfarkt und Subarachnoidalblutung.

10. Zubereitung gemäss irgendeinem der Ansprüche 1 bis 7 zur Verwendung bei der Verbesserung und Prävention von Arteriosklerose, Leberschädigung, Nierenschäden, Diabetes und Schädigung der Magen-Darm-Schleimhaut.

## Revendications

1. Préparation pour absorption percutanée contenant de la 3-méthyl-1-phényl-2-pyrazolin-5-one, **caractérisée en ce qu'**elle contient, comme principe actif, 0,1 à 30 pour cent en masse de 3-méthyl-1-phényl-2-pyrazolin-5-one représentée par la formule suivants : 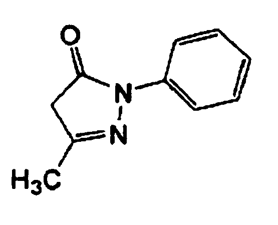 ou un sel de celle-ci acceptable au plan médical dans une base aqueuse, dans laquelle la base aqueuse contient, sur la base d'une quantité totale de la base aqueuse, 1 à 20 pour cent en masse d'un polymère hydrosoluble, 0,01 à 20 pour cent en masse d'un agent de réticulation, 10 à 80 pour cent en masse d'alcool polyhydrique, et 1 à 80 pour cent en masse d'eau.

2. Préparation adhésive pour absorption percutanée contenant de la 3-méthyl-1-phényl-2-pyrazolin-5-one, **caractérisée en ce qu'**un milieu support, une couche de base formée d'une base aqueuse contenant, comme principe actif, 0,1 à 30 pour cent en masse de 3-méthyl-1-phényl-2-pyrazolin-5-one représentée par la formule suivants : 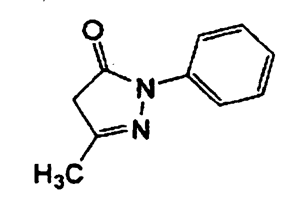 ou un sel de celle-ci acceptable au plan médical, et une couverture sont séquentiellement stratifiés et formés, dans laquelle la base aqueuse contient, sur la base d'une quantité totale de la base aqueuse, 1 à 20 pour cent en masse d'un polymère hydrosoluble, 0,01 à 20 pour cent en masse d'un agent de réticulation, 10 à 80 pour cent en masse d'alcool polyhydrique, et 1 à 80 pour cent en masse d'eau.

3. Préparation pour absorption percutanée contenant de la 3-méthyl-1-phényl-2-pyrazolin-5-one, **caractérisée en ce qu'**elle contient, comme principe actif, 0,1 à 30 pour cent en masse de 3-méthyl-1-phényl-2-pyrazolin-5-one représentée par la formule suivants : 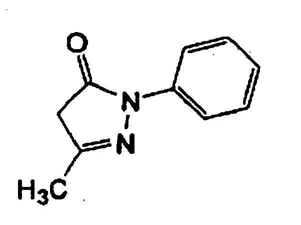 ou un sel de celle-ci acceptable au plan médical dans une base de caoutchouc, dans laquelle la base de caoutchouc contient, sur la base de la quantité totale de la base de caoutchouc, 10 à 50 pour cent en masse d'un polymère caoutchouc, 10 à 50 pour cent d'un plastifiant, et 5 à 50 pour cent en masse d'un agent collant.

4. Préparation adhésive pour absorption percutanée contenant de la 3-méthyl-1-phényl-2-pyrazolin-5-one, **caractérisée en ce qu'**un milieu support, une couche de base formée d'une base de caoutchouc contenant, comme principe actif, 0,1 à 30 pour cent en masse de 3-méthyl-1-phényl-2-pyrazolin-5-one représentée par la formule suivants : 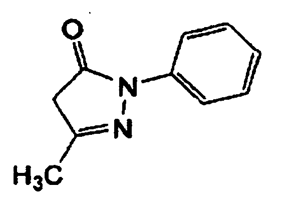 ou un sel de celle-ci acceptable au plan médical, et une couverture sont séquentiellement stratifiés et formés, dans laquelle la base de caoutchouc contient, sur la base de la quantité totale de la base de caoutchouc, 10 à 50 pour cent en masse d'un polymère caoutchouc, 10 à 50 pour cent d'un plastifiant, et 5 à 50 pour cent en masse d'un agent collant.

5. Préparation pour absorption percutanée selon la revendication 1 ou 2, **caractérisée en ce que** l'agent de réticulation est l'hydroxyde d'aluminium.

6. Préparation percutanée selon l'une quelconque des revendications 1 à 4, **caractérisée en ce que** la préparation contient en outre de la N-méthyl-2-pyrrolidone comme agent dissolvant.

7. Préparation pour absorption percutanée selon l'une quelconque des revendications 1 à 4, **caractérisée en ce que** la préparation contient en outre du crotamiton comme accélérant de l'absorption percutanée.

8. Préparation selon l'une quelconque des revendications 1 à 7 pour une utilisation dans la protection des fonctions cérébrales.

9. Préparation selon l'une quelconque des revendications 1 à 7 pour une utilisation dans l'amélioration et la prévention d'un dysfonctionnement cérébral, incluant un infarctus cérébral et une hémorragie sous-arachnoïdienne.

10. Préparation selon l'une quelconque des revendications 1 à 7 pour une utilisation dans l'amélioration et la prévention d'une artériosclérose, d'une lésion hépatique, d'une lésion rénale, d'un diabète et d'une lésion de la membrane muqueuse gastro-intestinale.
